# EUROPEAN PATENT SPECIFICATION

(11) **EP 2 044 952 B1**
(45) Date of publication and mention of the grant of the patent: **16.11.2011**
(21) Application number: 07735311.8
(22) Date of filing: 16.02.2007
(51) Int. Cl.: A61K 38/18, A61P 35/00, A61P 17/02

(54) **USE OF THE PEDF FACTOR TO INDUCE CELL REGENERATION**
VERWENDUNG DES PED-FAKTORS ZUR INDUKTION VON ZELLREGENERIERUNG
UTILISATION DU FACTEUR CELLULAIRE POUR INDUIRE LA RÉNOVATION DE CELLULES

(30) Priority: 17.02.2006 ES 200600386
(43) Date of publication of application: 08.04.2009
(73) Proprietor: Universitat De València, Estudi General, 46010 València (ES); Universidad de Castilla-La Mancha, 02071 Albacete (ES)
(72) Inventor: FARIÑAS GÓMEZ, Isabel, E-46010 Valencia (ES); ANDREU AGULLÓ, Celia, 46010 Valencia (ES); RODRÍGUEZ FERRÓN, Sacramento, 46010 Valencia (ES); RAMÍREZ CASTILLEJO, Carmen, 02071 Albacete (ES); SÁNCHEZ GÓMEZ, Pilar, 46010 Valencia (ES); MIRA APARICIO, Helena, 46010 Valencia (ES); ESCRIBANO MARTÍNEZ, Julio, 02071 Albacete (ES); SÁNCHEZ SÁNCHEZ, Francisco, 02071 Albacete (ES); AROCA AGUILAR, José Daniel, 02071 Albacete (ES)
(74) Representative: Illescas, Manuel
(86) International application number: PCT/IB2007/051115
(87) International publication number: WO 2007/093975

(56) References cited:
- WO-A-01/62725
- WO-A-2004/028559
- WO-A-2005/041887
- US-A1- 2004 096 750
- US-B2- 6 919 309

## Description

### FIELD OF THE INVENTION:

This invention belongs to the field of Biochemistry and Cell Biology. In particular, the invention refers to the specific use of the molecule PEDF (Pigmented Epithelium-Derived Factor) for the induction and/or potentiation of self-renewal in cultured stem cells. The present invention also refers to the use of this molecule for the activation of endogenous stem cells in different tissues by means of its exogenous administration to the corresponding tissue/organ. The invention also includes the use of pharmaceutical compounds containing at least an amount of PEDF that is pharmaceutically efficient for the stimulation of self-renewal and at least a pharmaceutically acceptable medium.

### BACKGROUND OF THE INVENTION:

The molecule PEDF (Pigmented Epithelium-Derived Factor) belongs to the serpin superfamily (serine protease inhibitors), although it lacks protease inhibiting activity. Human PEDF has 418 amino acids, a signal peptide for secretion and is N-glycosylated in Asn285. The secreted product exhibits an apparent molecular weight of 50 kDa. Two regions have been described in the PEDF molecule:
1) Asp44-Asn77 (34 amino acids), with anti-angiogenic activity,
2) Va178-Thr121 (44 amino acids), with neurotrophic activity.

PEDF is a multifunctional protein and it has previously been shown to be active as:
a) A stimulator of neural differentiation, in neuroblastoma cells.
b) A factor that reduces malignant phenotypes, in prostate tumour cells.
c) A stimulator of neuritic growth, in motor neurons.
d) A neuroprotective factor for neurons exposed to apoptotic stimuli, in retinal ganglion, cerebellar granular, hippocampal, and motor neurons.
e) A proliferation-blocking factor, in microglial cells.
f) An apoptosis-inducing factor, in tumour cells.
g) An apoptosis-inducing factor, in endothelial cells.
h) A migration-blocking factor, in endothelial cells.
i) An anti-angiogenic and anti-vasculogenic factor, in endotelial cells.

Thus, the molecule PEDF has been previously proposed as a potentially useful factor for the development, maintenance, and proper functioning of the retina as well as for the treatment of retinal degeneration, specially of the macula, induced by light, aging, diabetes, glaucoma, or genetic diseases such as retinitis pigmentosa, in which neovascularization is an aggravating factor *[*WO0224234*;* WO02058730*;* WO04028635*,* WO04027019*;* WO0181551*;* US020194639*;* US030087859*;* US010049369*;* Imai D, Yoneya S, Gehlbach PL, Wei LL, Mori K. (2005) Intraocular gene transfer of pigment epithelium-derived factor rescues photoreceptors from light-induced cell death. J Cell Physiol., 202(2):570-8 *;* Takita H, Yoneya S, Gehlbach PL, Duh EJ, Wei LL, Mori K. (2003) Retinal neuroprotection against ischemic injury mediated by intraocular gene transfer of pigment epithelium-derived factor. Invest Ophthalmol Vis Sci. 44(10):4497-504*;* Miyazaki M, Ikeda Y, Yonemitsu Y, Goto Y, Sakamoto T, Tabata T, Ueda Y, Hasegawa M, Tobimatsu S, Ishibashi T, Sueishi K. (2003) Simian lentiviral vector-mediated retinal gene transfer of pigment epithelium-derived factor protects retinal degeneration and electrical defect in Royal College of Surgeons rats. Gene Ther. 10(17):1503-11*.* Auricchio A, Behling KC, Maguire AM, O'Connor EM, Bennett J, Wilson JM, Tolentino MJ. (2002) Inhibition of retinal neovascularization by intraocular viral-mediated delivery of anti-angiogenic agents. Mol Ther., 6(4):490-4*. 5.* Mori K, Gehlbach P, Ando A, McVey D, Wei L, Campochiaro PA. (2002) Regression of ocular neovascularization in response to increased expression of pigment epithelium-derived factor. Invest Ophthalmol Vis Sci., 43(7):2428-34*. 6.* Raisler BJ, Berns KI, Grant MB, Beliaev D, Hauswirth WW. (2002) Adeno-associated virus type-2 expression of pigmented epithelium-derived factor or Kringles 1-3 of angiostatin reduce retinal neovascularization. Proc Natl Acad Sci U S A., 25;99(13):8909-14*.* Ogata N, Wang L, Jo N, Tombran-Tink J, Takahashi K, Mrazek D, Matsumura M. (2001) Pigment epithelium derived factor as a neuroprotective agent against ischemic retinal injury. Curr Eye Res. 22(4):245-52*.* Mori K, Duh E, Gehlbach P, Ando A, Takahashi K, Pearlman J, Mori K, Yang HS, Zack DJ, Ettyreddy D, Brough DE, Wei LL, Campochiaro PA. Pigment epithelium-derived factor inhibits retinal and choroidal neovascularization. J Cell Physiol., 188(2):253-63*.* Cao W, Tombran-Tink J, Elias R, Sezate S, Mrazek D, McGinnis JF. (2001) In vivo protection of photoreceptors from light damage by pigment epithelium-derived factor. Invest Ophthalmol Vis Sci. 42(7):1646-52*.* Stellmach V, Crawford SE, Zhou W, Bouck N. (2001) Prevention of ischemia-induced retinopathy by the natural ocular antiangiogenic agent pigment epithelium-derived factor. Proc Natl Acad Sci U S A., 98(5):2593-7*.* Cayouette M, Smith SB, Becerra SP, Gravel C. (1999) Pigment epithelium-derived factor delays the death of photoreceptors in mouse models of inherited retinal degenerations. Neurobiol Dis., 6(6):523-32*.* Cao W, Tombran-Tink J, Chen W, Mrazek D, Elias R, McGinnis JF. (1999) Pigment epithelium-derived factor protects cultured retinal neurons against hydrogen peroxide-induced cell death. J Neurosci Res., 57(6):789-800*]*

The molecule PEDF is also known as a potentially useful factor for neuroprotection in neurodegenerative processes or in cases of neuronal damage *[*WO0224234*;* WO02058730*;* WO04028635*;* WO04027019*;* WO0181551*;* US020194639*;* US030087859*;* US010049369*;* Cao W, Tombran-Tink J, Chen W, Mrazek D, Elias R, McGinnis JF. (1999) Pigment epithelium-derived factor protects cultured retinal neurons against hydrogen peroxide-induced cell death. J Neurosci Res., 57(6):789-800*.* Houenou LJ, D'Costa AP, Li L, Turgeon VL, Enyadike C, Alberdi E, Becerra SP. (1999) Pigment epithelium-derived factor promotes the survival and differentiation of developing spinal motor neurons. J Comp Neurol., 412(3):506-14*.* Bilak MM, Corse AM, Bilak SR, Lehar M, Tombran-Tink J, Kuncl RW. (1999) Pigment epithelium-derived factor (PEDF) protects motor neurons from chronic glutamate-mediated neurodegeneration. J Neuropathol Exp Neurol., 58(7):719-28*.* DeCoster MA, Schabelman E, Tombran-Tink J, Bazan NG. (1999) Neuroprotection by pigment epithelial-derived factor against glutamate toxicity in developing primary hippocampal neurons. J Neurosci Res., 56(6):604-10*.* Taniwaki T, Hirashima N, Becerra SP, Chader GJ, Etcheberrigaray R, Schwartz JP. (1997) Pigment epithelium-derived factor protects cultured cerebellar granule cells against glutamate-induced neurotoxicity. J Neurochem., 68(1):26-32*.* Taniwaki T, Becerra SP, Chader GJ, Schwartz JP. (1995) Pigment epithelium-derived factor is a survival factor for cerebellar granule cells in culture. J Neurochem. 1995 Jun;64(6):2509-17*.* Steele FR, Chader GJ, Johnson LV, Tombran-Tink J. (1993) Pigment epithelium-derived factor: neurotrophic activity and identification as a member of the serine protease inhibitor gene family. Proc Natl Acad Sci U S A. 1993 Feb 15,90(4):1526-30*].*

The molecule PEDF has also been described as an anti-tumor factor because it promotes differentiation of tumour cells and inhibits angiogenesis *[*US030082183*;* US040234505*;* W0050118136*;* US050222031*;* Cai J, Jiang WG, Grant MB, Boulton M. (2006) Pigment Epithelium-derived Factor Inhibits Angiogenesis via Regulated Intracellular Proteolysis of Vascular Endothelial Growth Factor Receptor 1. J Biol Chem., 281(6):3604-13*.* Streck CJ, Zhang Y, Zhou J, Ng C, Nathwani AC, Davidoff AM. Adeno-associated virus vector-mediated delivery of pigment epithelium-derived factor restricts neuroblastoma angiogenesis and growth. J Pediatr Surg. 40(1):236-43*.* Wang L, Schmitz V, Perez-Mediavilla A, Izal I, Prieto J, Qian C. (2003) Suppression of angiogenesis and tumor growth by adenoviral-mediated gene transfer of pigment epithelium-derived factor. Mol Ther., 8(1):72-9*.* Doll JA, Stellmach VM, Bouck NP, Bergh AR, Lee C, Abramson LP, Cornwell ML, Pins MR, Borensztajn J, Crawford SE. (2003) Pigment epithelium-derived factor regulates the vasculature and mass of the prostate and pancreas. Nat Med., 9(6):774-80*.* Guan M, Yam HF, Su B, Chan KP, Pang CP, Liu WW, Zhang WZ, Lu Y. (2003) Loss of pigment epithelium derived factor expression in glioma progression. J Clin Pathol., 56(4):277-82*.* Dawson DW, Volpert OV, Gillis P, Crawford SE, Xu H, Benedict W, Bouck NP. Pigment epithelium-derived factor: a potent inhibitor of angiogenesis. Science, 285(5425):245-8*].*

The molecule PEDF has also been proposed as a diagnosis factor for certain neurodegenerative diseases, such as amyotrophic lateral sclerosis in which PEDF levels in cerebrospinal fluid appear abnormally elevated, as well as a cancer prognosis factor, because its levels appear to inversely correlate with tumour progression *[*US050222031*;* Sidle DM, Maddalozzo J, Meier JD, Cornwell M, Stellmach V, Crawford SE. (2005) Altered pigment epithelium-derived factor and vascular endothelial growth factor levels in lymphangioma pathogenesis and clinical recurrence. Arch Otolaryngol Head Neck Surg., 131(11):990-5*.* Matsumoto K, Ishikawa H, Nishimura D, Hamasaki K, Nakao K, Eguchi K. Antiangiogenic property of pigment epithelium-derived factor in hepatocellular carcinoma. Hepatology, 40(1):252-9*.* Uehara H, Miyamoto M, Kato K, Ebihara Y, Kaneko H, Hashimoto H, Murakami Y, Hase R, Takahashi R, Mega S, Shichinohe T, Kawarada Y, Itoh T, Okushiba S, Kondo S, Katoh H. (2004) Expression of pigment epithelium-derived factor decreases liver metastasis and correlates with favorable prognosis for patients with ductal pancreatic adenocarcinoma. Cancer Res., 64(10):3533-7*.* Guan M, Pang CP, Yam HF, Cheung KF, Liu WW, Lu Y. (2004) Inhibition of glioma invasion by overexpression of pigment epithelium-derived factor. Cancer Gene Ther., 11 (5):325-32*.* Abe R, Shimizu T, Yamagishi S, Shibaki A, Amano S, Inagaki Y, Watanabe H, Sugawara H, Nakamura H, Takeuchi M, Imaizumi T, Shimizu H. (2004) Overexpression of pigment epithelium-derived factor decreases angiogenesis and inhibits the growth of human malignant melanoma cells in vivo. Am J Pathol., 164(4):1225-32*.* Miyagishi D, Ohno-Matsui K, Amagasa T, Morita I. (2003) Regulation of the expression of pigment epithelium-derived factor, an anti-angiogenic factor in human oral squamous cell carcinoma cell lines. Cancer Lett., 196(1):77-85*.* Guan M, Yam HF, Su B, Chan KP, Pang CP, Liu WW, Zhang WZ, Lu Y. Loss of pigment epithelium derived factor expression in glioma progression. J Clin Pathol., 56(4):277 82*.* Abramson LP, Stellmach V, Doll JA, Cornwell M, Arensman RM, Crawford SE. (2003) Wilms' tumor growth is suppressed by antiangiogenic pigment epithelium-derived factor in a xenograft model. J Pediatr Surg., 38(3):336-42*.* Slavc I, Rodriguez IR, Mazuruk K, Chader GJ, Biegel JA. (1997) Mutation analysis and loss of heterozygosity of PEDF in central nervous system primitive neuroectodermal tumors. Int J Cancer. 72(2):277-82*.* Seigel GM, Tombran-Tink J, Becerra SP, Chader GJ, Diloreto DA Jr, del Cerro C, Lazar ES, del Cerro M. (1994) Differentiation of Y79 retinoblastoma cells with pigment epithelial-derived factor and interphotoreceptor matrix wash: effects on tumorigenicity. Growth Factors, 10(4):289-97*.* Tombran-Tink J, Chader GG, Johnson LV. (1991) PEDF: a pigment epithelium-derived factor with potent neuronal differentiative activity. Exp Eye Res. 53(3):411-4*.* Kuncl RW, Bilak MM, Bilak SR, Corse AM, Royal W, Becerra SP. (2002) Pigment epithelium-derived factor is elevated in CSF. of patients with amyotrophic lateral sclerosis. J Neurochem., 81(1):178-84*].*

Nevertheless, the molecule PEDF has never been reported in the literature as a factor that can modulate the behavior of any kind of stem cell and, therefore, its potential use as a stimulator of stem cell self-renewal is novel and constitutes the object of the present invention. It is convenient to emphasize that the effect of PEDF on stem cell self-renewal claimed in the present invention is not at all related to any of the effects previously attributed to this molecule on cell processes, such as cell differentiation, survival, or apoptosis, in other cell types. Thus, the present invention provides a new use or application of the molecule PEDF as a factor that induces self-renewal in stem cell populations.

### DESCRIPTION OF THE INVENTION:

The present invention refers to the molecule PEDF for use in a method to enhance self-renewal of cultured stem cells by means of its exogenous administration. The addition of PEDF to defined culture media facilitates the maintenance of stem cells in the culture plate because PEDF stimulates: self-renewing symmetrical cell divisions vs. differentiative cell divisions Replication of cultured stem cells through differentiative divisions leads to the production of stem cell-derived progenitor cells with restricted potential and the consequent loss of stem cell potential. Thus, addition of PEDF leads to the improved expansion of stem cells and to the subsequent maintenance of stem cell potential.

The present invention includes the use of the molecule PEDF and/or of any derivative to promote the expansion of stem cell populations, adult or embryonic, of any species. This action is achieved by PEDF by promoting the replication of stem cells through self-renewing cell divisions as well as by inducing the expression of molecules that usually associate with an undifferentiated, multipotent, cell state. The invention contemplates the use of PEDF on naïve stem cells and on genetically and/or epigenetically modified stem cells. The invention also refers to pharmaceutically compounds containing a pharmaceutically efficient amount of PEDF for use in a method to stimulate endogenous somatic stem cells, for the enhanced *in situ* production of differentiated progeny.

The homogeneous expansion of a stem cell population in defined culture media *ex vivo* is not fully efficient because replication of these cells usually results in the production of derived progenitor cells that are capable of extensive proliferation but exhibit restrictions in self-rebewak and multipotency, properties that are characteristic of stem cells populations but that are lost in their derived progeny when stem cells divide in a non-renewing way. The observation that stem cell populations largely divide to produce restricted progenitors when expanded in culture suggests that culture conditions increase the probability of these cells to undergo differentiative divisions vs. self-renewing divisions Because the endogenous microenvironments ("niches") in which stem cells reside appear to provide a set of signals that balance the ratio between self-renewing divisions and differentiative divisions in a way that ensures lifelong persistence of the stem cell compartment, progressive loss of potential *in vitro* indicate that culture conditions do not recapitulate the natural microenvironment that these cells require to exhibit their full potential.

PEDF is found in neurogenic niches. PEDF is a secreted soluble molecule and, therefore, it can be easily added to culture media. PEDF can induce self-renewal of neural stem cells obtained from adult mice brains when added to the cultures, because it activates the self-renewing division of these cells and it induces the expression of molecules that are reported to be associated with undifferentiated, more multipotent, progenitor cells, such as transcritption factor Sox2 and Notch pathway effectors. When PEDF is added to neurospheres, which are floating aggregates formed by all the cell clonal progeny of a single neural stem cell *in vitro,* this factor increases the number of cells within each neurosphere capable of forming a new neurosphere when these are dissociated at the single cell level are re-plated at low density, i.e. 2.5 cells/microliter (self-renewing assay). In addition, expression of the undifferentiation marker Sox2 increases in neural stem cells treated with PEDF. Therefore, this invention also provides a method based on the enhancement of neural stem cell self-renewal by addition of PEDF to screen for possible inhibitors and/or antagonists of the PEDF effects or interactions.

In addition to the effects on the self-renewal of cultured neural stem cells, when PEDF is directly administered to adult mouse brains it causes the activation of endogenous neural stem cells in the subventricular zone, resulting in enhanced neurogenic activity and the subsequent production of more neurons. PEDF could, therefore, be a facilitating factor in endogenous processes of cell turnover from stem cells. Thus, the present invention also refers to the use of the molecule PEDF in the preparation of pharmaceutical compounds to activate endogenous stem cells.

The present invention claims the use of PEDF to induce self-renewing activation of any kind of stem cells independently of way in which the molecule is produced or obtained, as PEDF could be obtained directly from natural sources of produced by any artificial method. Thus, the present invention claims the use of any PEDF molecule, natural or synthetic, for the stimulation of stem cell self-renewal.

As a first important embodiment, the present invention refers to the use of the PEDF factor in its complete or fragmented sequence for manufacturing medicines for the activation of self-renewal in stem cells of any origin.

As another important embodiment, the present invention refers to the use of the factor PEDF for manufacturing medicines for the activation of self-renewal in stem cells of any origin which additionally comprise molecules selected from the group of: amino acids, lipids, carbohydrates and metals.

As another important embodiment, the present invention refers to the use of the factor PEDF for manufacturing medicines for the activation of self-renewal in stem cells of any origin after its production in or by prokaryotic or eukaryotic expression systems.

As another important embodiment, the present invention refers to the use of the PEDF factor in its complete or fragmented sequence for manufacturing medicines for the activation of self-renewal in stem cells of any origin, either cultured or endogenous stem cells.

As another important embodiment, the present invention refers to the use of the factor PEDF for manufacturing medicines for the activation of self-renewal in stem cells of any origin which are in a form selected from the group of: powder, granules, tablets, capsules, troches, solution, suspension, emulsion and syrup.

As another important embodiment, the present invention refers to the use of the factor PEDF for manufacturing medicines for the activation of processes of tissue regeneration in skin, nervous system, etc.

As another important embodiment, the present invention refers to the use of the factor PEDF for manufacturing medicines for their use in cell therapy, primarily in cell therapy approaches to cardiac, neural, and hematopoiteic regeneration.

As another important embodiment, the present invention refers to a pharmaceutically composition **characterized in that** it comprises, in a pharmaceutically acceptable medium, a pharmaceutically effective quantity of:
a) the total or partial sequence of the PEDF factor,
b) a biological natural variation of the PEDF factor, such as an allelic variant,
c) a functional equivalent of the PEDF factor which contains single or multiple substitution (s), addition (s), insertion (s), and or deletion (s) in the sequence of the wild type protein and/or substitutions with chemically modified amino acids which do not affect its biological function,
d) an active fragment of the PEDF factor which comprises a partial sequence of the PEDF factor that keeps its biological function,
e) a fusion protein which comprises the PEDF factor fused to a peptide or to other protein,
f) a structural analogue of the PEDF factor,
g) and/or a homologue of the PEDF factor.

As another important embodiment, the present invention refers to a pharmaceutically composition that it contains at least a pharmaceutically effective quantity of the total or partial sequence of the PEDF factor and at least a pharmaceutically acceptable carrier.

As another important embodiment, the present invention refers to a pharmaceutically composition that it contains at least a pharmaceutically effective quantity of a biological natural variant of the PEDF factor and at least a pharmaceutically acceptable carrier.

As another important embodiment, the present invention refers to a pharmaceutically composition that it contains at least a pharmaceutically effective quantity of a functional equivalent of the PEDF factor and at least a pharmaceutically acceptable carrier.

As another important embodiment, the present invention refers to a pharmaceutically composition that it contains at least a pharmaceutically effective quantity of an active fragment of the PEDF factor and at least a pharmaceutically acceptable carrier.

As another important embodiment, the present invention refers to a pharmaceutically composition that it contains at least a pharmaceutically effective quantity of a fusion protein of PEDF factor and at least a pharmaceutically acceptable carrier.

As another important embodiment, the present invention refers to a pharmaceutically composition that it contains at least a pharmaceutically effective quantity of a PEDF factor derivative and at least a pharmaceutically acceptable carrier.

As another important embodiment, the present invention refers to a pharmaceutically composition that it contains that it contains at least a pharmaceutically effective quantity of at least a PEDF factor structural analogue and at least a pharmaceutically acceptable carrier.

As another important embodiment, the present invention refers to a pharmaceutically composition that it contains that it contains at least a pharmaceutically effective quantity of at least a PEDF factor homologue and at least a pharmaceutically acceptable carrier.

As another important embodiment, the present invention refers to a pharmaceutically composition **characterized in that** the PEDF factor quantity is at least 20 ng/ml.

In cell regeneration processes following lesion (cardiac, skin, or neural tissues after vascular accident or trauma) or in pathological situations (hematopoietic, following bone marrow transplant; neural, in neurodegenerative diseases) increased numbers of cells are needed for tissue repair. This process could be induced/favoured if we are capable of activating endogenous stem cells in adult tissues by an acceleration of the physiological homeostatic process of cell turnover. This activation should activate stem cells for the production of demanded cell progeny without exhausting the stem cell pool, that is, maintaining self-renewal. On the other hand, the cell therapy approach is based on the expansion *ex vivo* of stem cell populations to subsequently drive their differentiation to required cell phenotypes that will be transplanted into the lesioned structure for repair. Ways are needed to preserve full potential of cultured stem cell populations to ensure that differentiated progeny of the desired cell types can be obtained after stem cell expansion.

In the context of the present invention, a partial sequence of PEDF refers to any fragment of the PEDF molecule including a number of amino acids lower than the number of amino acids of the natural full length PEDF molecule and to any synthesized peptide with a sequence identical or similar to any part of the PEDF molecule.

In the context of the present invention, a natural biological variant of PEDF refers to all PEDF molecules from any species or bearing natural amino acid substitutions, or to those PEDF molecules produced in recombinant form in different species and that are modified in different ways.

In the context of the present invention, a functional equivalent to PEDF refers to all total or partial sequences of PEDF that have been obtained after directed mutagenesis or that have been modified by the addition of chemical groups or atoms to change its stability, diffusion rate, permeability, or biological activity.

In the context of the present invention, an active fragment of PEDF refers to any peptide sequence of any number of amino acids that retains the function of the full length PEDF molecule.

In the context of the present invention, a fusion protein refers to PEDF fused to a peptide or to another protein, as well as to a PEDF molecule that could include a set of amino acids (tag) at either end that can aid in the purification, tracing, or in interaction assays.

In the context of the present invention, a structural analogue refers to a molecule that has similar, though not identical, structure to PEDF and similar activity.

In the context of the present invention, a PEDF homologue refers to a molecule that has similar, though not identical, sequence to PEDF and similar activity.

### DESCRIPTION OF THE FIGURES

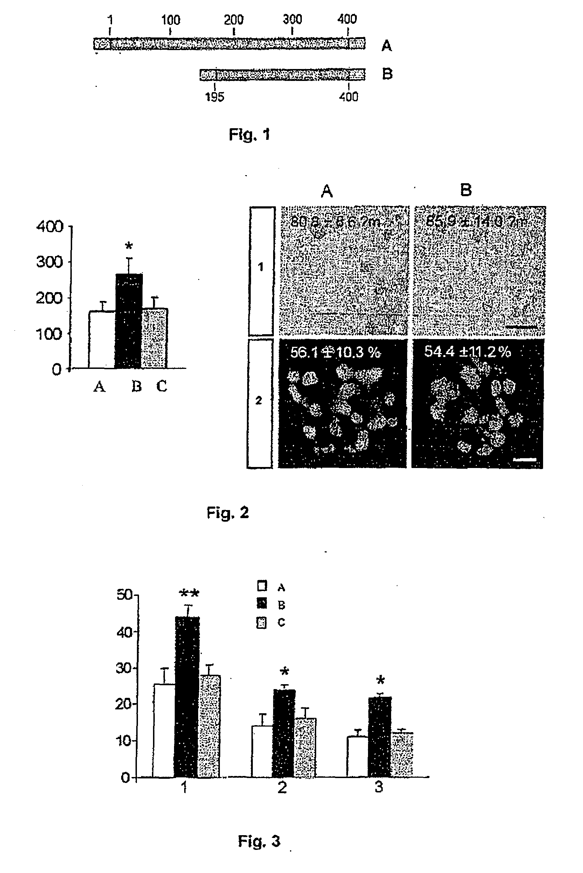

**Fig. 1**. This figure shows a simple schematic drawing of the human full length polypeptide PEDF (A) and of a truncated form, which contains the anti-angiogenic but not the neurotrophic domain, comprising from amino acid 195 to amino acid 400 (that we denominate "C-ter PEDF") (B).
**Fig. 2**. Effect of human PEDF and C-ter PEDF produced in *Pichia pastoris* on neurosphere cultures derived from the subventricular zone (SVZ) of adult mouse brains. In the histogram the X axis corresponds to the number of neurospheres formed, A corresponds to Control, B corresponds to PEDF, C corresponds to C-ter PEDF. In the picture panel, 1 corresponds to phase contrast optics and the numerical values indicate neurosphere mean diameters (mean ± s.e.m), 2 corresponds to immunocytochemical detection of the proliferation marker BrdU and the numerical values indicate percentages of cells that incorporate BrdU relative to the total numbers of cells (men ± s.e.m). Asterikc indicates level of statistical significance in a Student's t-test: (*p < 0.05).
**Fig. 3****.** Neurospheres grown in the presence of PEDF (the truncated form of PEDF, C-ter PEDF, is the control and has no effect in this assay) were dissociated and re-plated in different mitogenic conditions but in the absence of PEDF. In this figure, A corresponds to control (normal growth in EGF and FGF), B corresponds to pre-treatment with PEDF, and C correspond to pre-treatment with C-ter PEDF. In the histogram, 1 corresponds to EGF plus FGF, 2 corresponds to EGF, and 3 corresponds to FGF. The Y axis represents the number of neurospheres formed. Asterisks indicate level of statistical significance in a Student's t-test: (*p < 0.05; **p < 0.01).

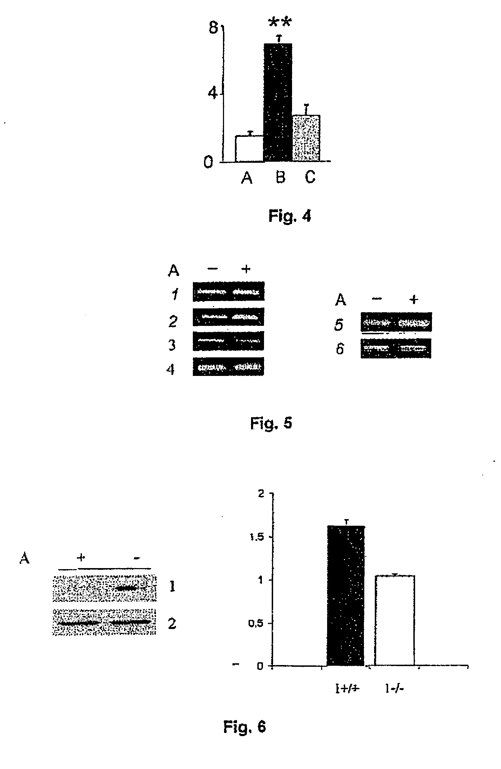

**Fig. 4****.** The histogram shows that after PEDF treatment the percentage of LeX+ (a marker which correlates with the most multipotent population of neural stem cells) cells in the neurospheres was increased. A corresponds to the control, B corresponds to PEDF treatment and C corresponds to C-ter PEDF treatment. X axis corresponds to the increase in the number of LeX+ cells, determined by cytometry, relative to the control condition A. Asterisks indicate level of statistical significance in a Student's t-test: (**p<0.01).
**Fig. 5****.** Changes induced by PEDF in the mRNA levels of several genes that are considered as undifferentiation markers, as determined by RT-PCR. A corresponds to PEDF, (-) corresponds to untreated samples and (+) corresponds to treated samples, 1 corresponds to Hes 1, 2 corresponds to Hes5, 3 corresponds to Mash1/Ascl1, 4 corresponds to β-actin, 5 corresponds to Sox2 and 6 corresponds to β-actin.
**Fig. 6****.** PEDF increases the expression of the cell cycle regulator p21, as determined by western-blot with specific anti-p21 antibodies. This increase in p21 levels is necessary for PEDF action since neural stem cell cultures derived from p21 deficient SVZ exhibit no changes in neurosphere number after PEDF treatment. A corresponds to PEDF, (-) shows untreated samples and (+) shows treated samples, 1 corresponds to p21, 2 corresponds to β-actin. In the histogram, 1+/+ correspond to neurospheres obtained from wild-type mice and 1-/- to those obtained from p21 deficient animals. Y axis represents the increase in the number of spheres induced by PEDF relative to the increase obtained with FGF and EGF alone (taken as the basal value of 1).

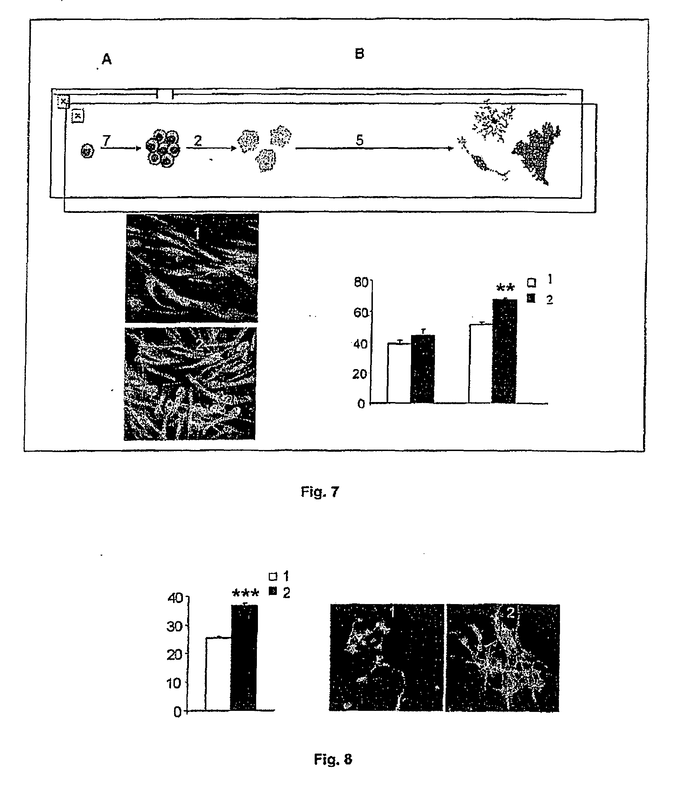

**Fig. 7****.** Treatment with PEDF increases the number of cells expressing cell undifferentiation markers *in vitro* (nestin), without affecting their proliferation. In the picture panels, immunocytochemical detection of antigens nestin (cytoplasmic) and BrdU (nuclear) are shown. In the histogram, Y axis corresponds to percentage of cells marked with BrdU (left bars) and nestin (right bars) over the total number of cells in the well plate. 1 corresponds to control, 2 corresponds to PEDF. Asterisks indicate level of statistical significance in a Student's t-test after arcsen (relative value) data transformation (**p < 0.01).
**Fig. 8****.** Treatment with PEDF improves the neurogenic capacity of neurosphere cultures. In the histogram, 1 corresponds to control and 2 corresponds to PEDF. Y axis shows the percentage of cells that have differentiated to neurons relative to the total number of cells in the plate. Asterisks indicate level of statistical significance in a Student's t-test (***p < 0.001), In the picture panels that show immunodetection of the neuronal antigen β-III-tubulin, 1 corresponds to control and 2 corresponds to PEDF.

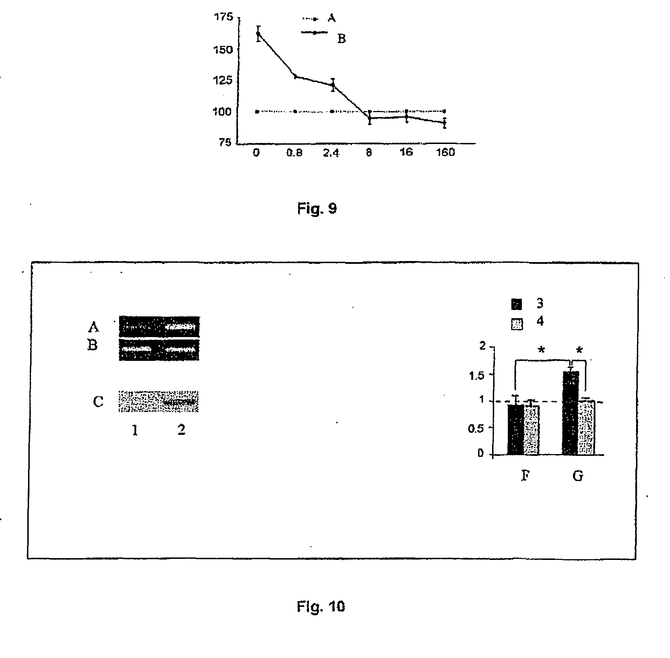

**Fig. 9****.** The truncated form containing the half C-terminal PEDF polypeptide antagonizes the PEDF full length action in the self-renewal assay. In the graph, the dotted line with symbol ■ represents control (A) and the continuous line with symbol ● represents treatment with 0.4 nM PEDF along with increasing concentrations of C-ter PEDF (B) shown in axis X (in nM). Y axis represents the number of spheres formed in each condition.
**Fig. 10****.** Transfected COS-7 cells with a pcDNA3.I-human PEDF construct express and secrete active PEDF as show in a self-renewal assay with neural stem cells and this effect is blocked by the C-ter PEDF. In the left panel A corresponds to RT-PCR detection of Pedf mRNA (Serpinf1), B corresponds to RT-PCR detection of β-actin mRNA, C corresponds to western blot analysis of PEDF protein in the COS-7 conditioned media, 1 corresponds to COS-7 cells transfected with empty vector (pcDNA3.1), 2 corresponds to COS-7 cells transfected with pcDNA3.1-human PEDF construct. In the histogram, 3 corresponds to co-cultures with transfected COS-7 cells, 4 corresponds to the same conditions with C-ter PEDF which blocks the effects, F corresponds to COS-7+pcDNA (empty vector, control), G corresponds to COS-7+pcDNA-Pedf. Y axis corresponds to the increase in the number of neurospheres relative to control.

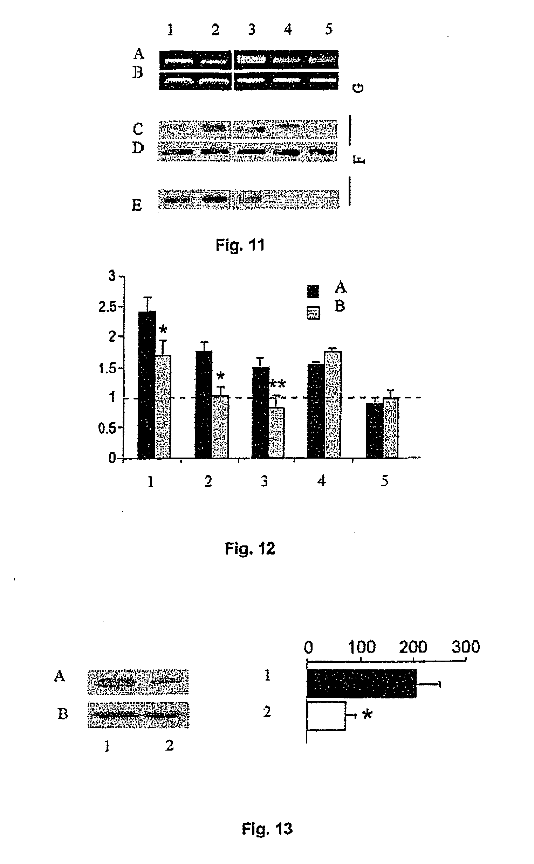

**Fig. 11****.** Endothelial and ependymal cells express and secrete PEDF while other cell types can express Pedf mRNA but do not secrete detectable quantities of factor. A corresponds to Pedf mRNA, B corresponds to RT-PCR detection of β-actin mRNA, C corresponds to PEDF protein, D corresponds to β-tubulin protein from cellular lysates, E corresponds to PEDF protein determined by western blot in conditioned media obtained from different cell types. F corresponds to western blot, G corresponds to RT-PCR. The cell types tested were human venous endothelial cells (HUVEC) (1), human arterial endothelial cells (HUAEC) (2), ependymal cells from the subventricular zone (3), astrocytes (4), and cerebellar granule neurons (5).
**Fig. 12****.** Endothelial and ependymal cells that express and secrete PEDF induce neurosphere formation and this induction effect is blocked by 15 nM C-ter PEDF. In this figure, A corresponds to co-cultures with different cell types, B corresponds to the same conditions than in A but in the presence of C-ter PEDF that blocks the full length PEDF effects. The cell types tested were human venous endothelial cells (HUVEC) (1), human arterial endothelial cells (HUAEC) (2), ependymal cells from the subventricular zone (3), astrocytes (4), and cerebellar granule neurons (5). Y axis corresponds to the increment in neurosphere number formed relative to the number of neurospheres obtained in the control condition with EGF and FGF. The statistical significance of the C-ter blockade is indicated. Asterisks indicate level of statistical significance in a Student's t-test after arcsen (relative value) data transformation (*p < 0.05; **p < 0.01).
**Fig. 13****.** Silencing of PEDF expression in HUVEC cells with a specific siRNA resulted in decreased PEDF secretion, and in a reduction in the number of neurospheres formed from co-cultured neural stem cells, than in HUVEC treated with a siRNA control. In the left panel, A corresponds to detection of PEDF in cell lysates by western blot, B corresponds to the detection of β-actin protein in the same lysates. 1 corresponds to HUVEC cells transduced with control siRNA, 2 corresponds to HUVEC cells transduced with a specific siRNA for Pedf. Y axis corresponds to the number of neurospheres formed. Asterisks indicates level of statistical significance in a Student's t-test (*p < 0.05).

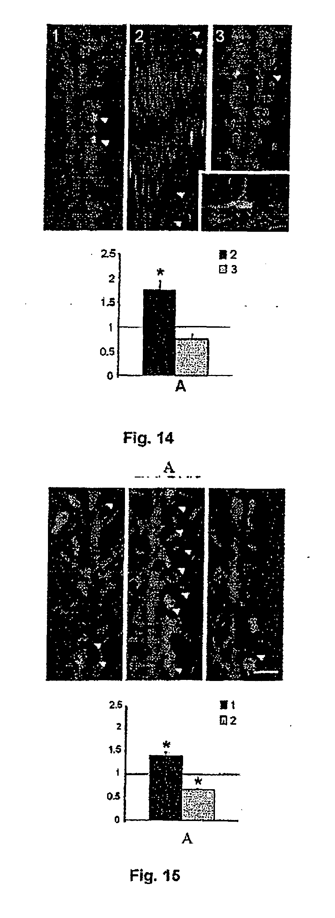

**Fig. 14****.** Intracerebral administration of PEDF increments the number of neural stem cells, but blockade of endogenous PEDF does not result in changes in their numbers. Pictures show immunodetection of long-term BrdU over DAPI staining in sections through the subventricular zone. Insert shows that long-term BrdU-positive cells are GFAP+ (cytoplasm and cell processes), an indication that they are neural stem cells of this region. 1 corresponds to saline, 2 corresponds to PEDF, 3 corresponds to C-ter PEDF, 4 corresponds to BrdU^{L}/GFAP. In the histogram, the Y axis corresponds to the relative increase in the number of BrdU retaining cells (A) after intraventricular PEDF infusion (2) or C-ter PEDF infusion (3) relative to saline infusion (control value=1). Asterisks indicates level of statistical significance in a Student's t-test after arcsen (relative value) data transformation (*p < 0.05; **p < 0.01).
**Fig. 15****.** Intracerebral administration of PEDF at subventricular zone level activates neural stem cell divisions. Pictures show immunodetection short-term BrdU (nuclear) and staining for GFAP (cytoplasm and cell processes). 1 corresponds to saline, 2 corresponds to PEDF, 3 corresponds to C-ter PEDF. In the histogram, the Y axis corresponds to the relative increase in the number of BrdU retaining cells (A) after intraventricular PEDF infusion (2) or C-ter PEDF infusion (3) relative to saline infusion (control value = 1). Asterisks indicates level of statistical significance in a Student's t-test after arcsen (relative value) data transformation (*p < 0.05).

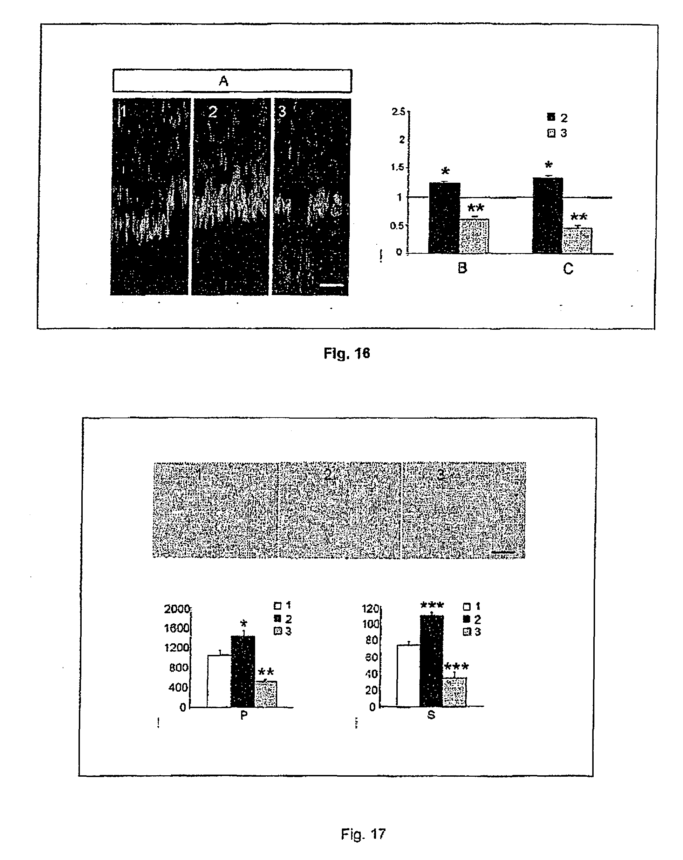

**Fig. 16****.** Intracerebral administration of PEDF at subventricular zone level actives endogenous neurogenesis. Pictures (A) show immunostaining for short-term BrdU (nuclear) and for PSA-NCAM (cytoplasm and cell processes). 1 corresponds to saline, 2 corresponds to PEDF, 3 corresponds to C-ter PEDF. In the histogram, Y axis corresponds to the relative increase in the number of BrdU retaining cells (B) or in cells that stain with anti-PSA-NCAM antibodies (C) in brains infused with PEDF (2) or C-ter PEDF (3) relative to those infused with saline (control value = 1). Asterisks indicates level of statistical significance in a Student's t-test after arcsen (relative value) data transformation (*p < 0.05; **p < 0.01).
**Fig. 17****.** Intracerebral administration of PEDF induces a higher production in neurospheres obtained from infused brains. Pictures show neurospheres under phase contrast optics. 1 corresponds to saline, 2 corresponds to PEDF, 3 corresponds to C-ter PEDF. In the histogram at the left, Y axis corresponds to the number of primary neurospheres formed from saline infused brains (1), PEDF infused brains (2) or C-ter infused brains (3). In the histogram at the right, Y axis corresponds to the number of secondary neurospheres formed from saline (1), PEDF (2) or C-ter (3) infused brains. Asterisks indicates level of statistical significance in a Student's t-test after arcsen (relative value) data transformation (*p < 0.05; **p < 0.01; ***p < 0.001).

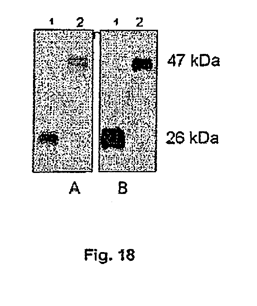

**Fig. 18****.** Purified forms of full-length and C-terminal half of the human PEDF obtained from *Pichia pastoris* medium analyzed by SDS-PAGE electrophoresis, stained with Coomasie blue (A) and immunodetected with anti-PEDF antibodies (B). 1 corresponds to PEDF (47 kDa) and 2 corresponds to C-ter PEDF (26 kDa).

### EXAMPLES:

### Example 1: Self-renewal assay for neural stem cells in culture

The PEDF peptides used for the assays are shown in Figure 1, This figure contains a schematic drawing showing the structure of the human full length PEDF and of a truncated form of PEDF (C-ter PEDF) which contains the anti-angiogenic but not the neurotrophic domains and that comprises amino acids 195-400, Full length PEDF regulates stem cell self-renewal. C-ter PEDF was used as a negative control because it is unable to promote self-renewal. In further experiments. C-ter PEDF will be used as a competitor of the full length PEDF.

We have demonstrated by the self-renewal assay that PEDF is able to promote self-renewal *in vitro* of the adult neural stem cells obtained from subventricular zone. The treatment of neurospheres (which are clonal agregates composed by the progeny of the neural stem cell that initiated the clone) With PEDF increases the number of cells that are able to form new spheres/clones when they are re-piated at a density of 2.5 cell/µl (self-renewal assay) (see Figure 2). In Figure 2 it is shown how the addition of PEDF to a neural stem cell culture promotes the formation of more neurospheres relative to the number of clones grown in complete medium (their usual culture medium with growth factors EGF and EGF). The addition of PEDF does not modify the size of the neurosphere (see average diameter of neurospheres) or the BrdU incorporation These results indicates that PEDF is not a classical mitogen.

In order to obtain adult neural stem cell cultures, mice from 2 to 4 months were sacrified by cervical dislocation Brains were extracted and washed in cold sterile PBS. The subventrular zone was dissected out excluding the striatal area and choroid plexuses. The pieces of dissected tissue were incubated with an enzymatic solution for 30 minutes at 37 °C, and afterwards, the tissue was dissociated with a fired polished Pasteur pipette until a homogeneous cellular suspension was obtained. The cells were seeded in complete medium (defined culture medium with 20 ng/ml EGF (Epidermal Growth Factor; invitrogen) and 10 ng/ml FGF2 (Fibroblast Growth Factor. Sigma)),[*Control medium*: DMEM/F12 supplemented with 0,6 % glucose. 0.1 % NaHCO₃. 5 mM HEPES, 2 mM L-glulamine (Invitrogen), 50 units/ml peniciline/streptomicine (Invitrogen), 0.08 mg/ml apo-t-transferrine (Sigma), 0.02 mg/ml insuline (Sigma), 9 µg/ml putrescine (Sigma), 16 nM progesterone (Sigma) y 24 nM Na₂SeO₃ (Sigma). *Complete medium:* control medium supplemented with 4 mg/ml BSA (bovine serum albumin, Sigma), 0.7 units/ml heparine (Sigma), 20 ng/ml EGF and 10 ng/ml FGF2] and grown at 37 °C in a 5% CO₂ atmosphere until the size of neurospheres appropiated. Cells cultured for approximately 6 DIV were dissociated to single cells, re-plated in fresh complete medium where they formed new neurospheres. The culture could be maintained for long periods of time by periodic passaging. For the self-renewal assay, dissociated cells were seeded at a density of 2.5 cell/µl and treated with PEDF or C-ter PEDF at 20 ng/ml. After 4-6 DIV the number of neurospheres formed was counted and the diameter of the clones measured. For BrdU incorporation analysis, a BrdU solution (2 µM) was added to a 48 hr-culture for 5 minutes. After that, the spheres were attached to cover slides pre-treated with poly-D-lysine at 10 µg/ml and fixed with 4 % paraformaldehyde for 20 minutes followed by 3 washes with 0.1 M PB. The immunocytochemical detection of the BrdU incorporated to the cultures was done with specific antibodies.

Neurospheres treated with PEDF contain more sphere-forming cells at low density, a property of neural stem cells with high potential (Figure 3). In Figure 3 an neurosphere assay of clones/neurospheres pre-treated with PEDF (the PEDF truncated form, named C-ter PEDF, is used as the control without effect). When the clones are dissociated and re-plated in the absence of PEDF more new clones are observed in any mitogenic condition (increased capacity of forming spheres).This result indicates that treatment of cells with PEDF induced self-renewal divisions when PEDF was previously added to the culture and that, therefore, PEDF promotes the generation of new sphere-forming cells through self-renewing divisions.

To determine if the increased capacity to form new spheres (secondary spheres) was related to the presence of more multipotent stem cells in PEDF-treated cultures, we determined the presence in these cultures of more cells expressing the surface antigen (LeX), a marker reported to be associated with the more multipotent fraction of neural stem cells (see Figure 4). In Figure 4 it is shown how the treatment with PEDF increases the number of cells within the neurospheres expressing LeX. To analyze the presence of this surface antigen (LeX), neurospheres were collected by centrifugation after 6 DIV and mechanically dissociated until a homogeneous cellular suspension was obtained. The cells were re-suspended in control medium (without mitogens) containing anti-LeX/SSEA-1 monoclonal antibody from Developmental Studies Hybridoma Bank (University of lowa, USA) for 15 minutes at 37 °C. After that, cells were fixed with 4 % PFA for 10 minutes at 4 °C, washed and incubated with mouse biotinylated anti-IgM antibody (1:200) followed by Cy3-conjugated streptavidine (1:1000, Jackson Laboratories). The cells were analyzed by flow cytometry with at least 20,000 cells for culture and the percentage of LeX positive cells was determined. The analysis was done with a Beckman Coulter Epics analyzer with a 488 nm argon laser.

### Example 2: Assay of the induction of multipotency in neural stem cells in culture at the molecular level

The treatment of neural stem cells with PEDF increases the number of neurosphere-forming cells and this effect is accompanied by molecular changes in the cells that are related to a more undifferentiated state, a necessary property for the maintenance of multipotency. Treatment with PEDF increases the expression of molecular effectors of Notch signalling, i. e. Hes1 and Hes5, which inhibit neuronal differentiation. Similarly, PEDF increases the expression of a factor associated to a more undifferentiated neural state. In Figure 5 it is shown how the levels of some undifferentiation markers are induced by PEDF. RT-PCR analyses were carried out to evaluate the expression of different genes. For these experiments total RNA was extracted with Rneasy Mini Kit (Qiagen) following manufacturer instructions. 1×10⁶ cells grown in complete medium with or without PEDF were collected. RT-PCR analyses were done using 1 µg of RNA retro-transcripted to cDNA using random primers. (3 µg/µl, Invitrogen Life Technologies, USA) and 200 U of Reverse Transcriptase SuperScript 11 RT (Invitrogen Life Technologies), in a total reaction volume of 40 µl and in the presence of first strand buffer (50 mM Tris-HCl, 75 mM KCl, 3 mM Mg₂Cl, 5 mM DTT and 0.25 mM dNTPs (Amersham Pharmacia). PCR reactions were carried out in an Eppendorf thermocycler using 1 µl de cDNA as template, in a reaction volume of 20 µl containing 4 mM Tris-HCl, 20 mM KCl, 20 µM EDTA, 200 µM DTT, 0.25 mM dNTP_{S} (Amersham Pharmacia), 0.25 µM primers (Sigma-Genosys) and 1 U de DNA Taq polimerase (Eppendorf, Hamburg, Alemania). Initial denaturation step at 94°C for 2 minutes was followed by corresponding amplifying cycles for each primer pair and by a final elongation step at 72 °C for 10 minutes. The PCR product was resolved in a 2% agarose gel electrophoresis with TBE buffer (89 mM Tris, pH 8.0, 89 mM Boric acid, 2mM EDTA) with 10µg/ml ethidium bromide. The loading buffer (6x) contained 50% glycerol, 0.05% bromophenol blue and 100 mM EDTA. The analysis of the bands was performed by densitometry comparing every band intensity to the beta-actin levels. Primers used were: [mHes1 (CGGTCTACACCAGCAACAGT;CACATGGAGTCCGAAGTGAG),mHes5(GCAGCATA GAGCAGCTGAAG;GAAGGCTTTGCTGTGTTCA),mSox2(ATGCACAACTCGGAGATC AG;TATAATCCGGGTGCTCCTTC), mβ-actin (CCGGGACCTGACAGACTACCT; GCCATCTCCTGCTCGAAGTCTA). As shown in Figure 5, PEDF increases the levels of the transcription factor Sox2, related to an undifferentiated state. The invention provides an assay to evaluate self-renewal in neural stem cells by the exogenous addition of PEDF and to screen for inhibitors of this process.

Self-renewal of neural-stem cells is related to molecules that regulate cell cycle. Expression of p21waf1, which belongs to the Cip/Kip family of cyclin dependent kinase inhibitors, is necessary for the persistence of somatic stem cells and for their unlimited self-renewal. In figure 6, it is shown that PEDF increases the expression of p21 using western-blot analysis with specific antibodies. This increase in p21 levels is necessary for PEDF effects because PEDF-treated cells from p21 knock-out mice do not produce more clones/neuorspheres, as shown in Figure 6. p21-deficient mice were obtained from the laboratory of Phillip Leder (Department of Genetics, Harvard Medical School, Boston, Massachusetts) and the mutation is in a C57BL6/129 background and wild-type mice were obtained from Jackson laboratories (Bar Harbor, ME). The mutant mice were maintained in homozygosis. Genotyping was performed by PCR analysis of genomic DNA obtained from a tail fragment. Tissue was digested in lysis buffer (100 mM Tris, pH 8.0, 200 mM NaCl, 2% SDS, 5 mM EDTA, 200 µg/ml proteinase K) at 55 °C for 12 hours. After enzymatic digestion, cell lysates were centrifuged at 14,000 rpm, 10 minutes, and the supernatant transferred to clean tubes. Afterwards, 200 µl of chloroform (Sigma) and 0.2 volumes of 8 M potassium Aacetate (Panreac) were added to the tubes, and these were shaked and incubated at -80 °C for 1 hour. After this time the tubes were thawed at room temperature and centrifuged at 14,000 rpm for 15 minutes to facilitate the separation between aqueous and organic phase. 200 µl of the aqueous phase were transferred to a clean tube and the genomic DNA was precipitated with 1 ml of absolute ethanol. The tubes were centrifuged at 14,000 rpm for 10 minutes, and the pellets of genomic DNA were washed with 70% ethanol. DNA was dried and re-suspended in 100 µl milliQ H₂O. Primers used were: p21+116F (AAGCCTTGATTCTGATGTGGGC), p21-135(TGACGAAGTCAAAGTTCCACCG) and p21-Neo19+(GCTATCAGGACATAGCGTTGGC) corresponding to *neo* sequence. Amplification steps are listed: denaturation step at 94 °C for 50 seconds, annealing at 55 °C for 30 sec, and elongation at 72 °C for 2 min. After a 40 cycle-reaction the wild-type allele ( 0.7 kbp) and the mutant allele (0.5 kbp) were resolved in agarose gels.

### Example 3: Induction assay for neural stem cell undifferentation

PEDF promotes an undifferentiated state in neural stem cells. PEDF treatment of neural stem cells that are plated for differentiation in an adhesive substrate and in the absence of mitogens results in a more undifferentiate phenotype that lasts longer. Treated cultures have more cells expressing the undifferentiation cell marker nestin (Figure 7). In Figure 7 it is shown how PEDF treatment increases the number of cells expressing nestin in the cultures, without affecting their proliferation. For the induction of differentiation, neurospheres grown for 5 DIV in presence or absence of PEDF were collected and washed several times with control medium to eliminate mitogen and PEDF traces. Cells were seeded in pre-treated wells (for 2 hours with Matrigel, 15 mg/ml stock solution diluted at 1:100 in control medium, Becton Dickinson, Bedford, MA) at a density of 1×10⁴ cells/ml in a differentiation medium containing control medium supplemented with FGF (20 ng/ml), a growth factor that promotes cellular survival in the first steps of the differentiation. At 2 DIV, cells were treated with BrdU (2 µM) for 5 minutes and fixed with 4 % PFA for 20 minutes. After several washes with 0.1 M PB, immunocytochemical detection of nestin and of BrdU incorporation was performed using nestin- and BrdU-specific antibodies. To do so, unspecific binding was blocked using a blocking buffer that contained 10% goat serum (Invitrogen), 10% (p/v) glycine and 0.25% (v/v) Triton-X-100 as a non-ionic detergent that permeabilizes the cells. The cells were incubated overnight at 4°C with a monoclonal anti-nestin antibody from DSHB (University of lowa, USA) and a polyclonal anti-BrdU antibody (Dako) diluted in the same blocking buffer. Cells were washed 3 times with PB 0.1M afterwards and were incubated with fluorescent secondary antibodies: goat anti-rabbit Alexa 350 (1:200), goat anti-mouse Cy3 (1:2000) followed by a bisbencimide nuclear (DAPI) counterstaining.

### Example 4: Neurogenesis induction assay in cultured neural stem cells

Nevertheless, PEDF treatment during the expansion phase does not block the terminal differentiation of these cells into neurons. In fact, as shown in Figure 8, neurogenesis is potentiated in PEDF treated cultures. Figure 8 shows that the increase in undifferentiation markers does not impede the terminal differentiation of the cultures after the time in vitro at which final differentiation of the culture is normally culminated. Moreover, an increase in the neurogenic capability is observed after pretreatment with PEDF, favoring the differentiation of a higher number of neurons (Tuj1-positive). 2 DIV, after seeding the NSCs onto the adherent substrate, as mentioned in the previous paragraph, medium was changed for control medium supplemented with 2% fetal bovine serum (Invitrogen) for 5 additional DIV, which favors neuronal and glial maturation. At 7 DIV, cells were fixed with 4% PFA prepared in PB 0.1M during 20 min at room temperature and were evaluated by immunocitochemistry to detect specific markers for neurons and glial cells, and the proportion of each of the phenotypes was determined. Neuronal and glial differentiation was defined using a triple immunostaining with a primary antibody anti-Tuj1 (neurons), anti-GFAP (astrocytes) and anti-MBP (oligodendrocytes). After cell fixation and several washes in PB 0.1 M, unspecific binding was blocked using a blocking buffer that contained 10% goat serum (Invitrogen), 10% (p/v) glycine and 0.25% (v/v) Triton-X-100 as a non-ionic detergent that permeabilizes the cells. The cells were incubated overnight at 4 °C with the following primary antibodies diluted in the same blocking buffer: mouse monoclonal (IgG) anti-βlll-Tubulin (Tuj1) (1:300; Covance); rabbit polyclonal (IgG) anti-glial fibrillary acidic protein (GFAP) (1:300; Dako); rat monoclonal (IgG) anti-myelin basic protein (MBP) (1:300, Chemicon). Cells were washed 3 times with PB 0.1M afterwards and were incubated with the following fluorescent secondary antibodies: goat anti-rabbit Alexa 350 (1:200), goat anti-mouse Cy3 (1:2000), biotinilated goat anti-rat (1:300), Cy2-conjugated streptoavidin (1:200) followed by a bisbencimide nuclear staining (DAPI). Bulk differentiation was used to determine the neurogenic potential of the different cultures. Cover glasses were mounted onto a microscope slide with a permanent mounting medium to protect fluorescence (Fluorsave, Calbiochem, La Jolla, CA) and the number of neurons, astrocytes and oligodendrocytes was calculated counting the number of neurons and astrocytes, or oligodendrocytes, *versus* the total number of DAPI stained nuclei in at least 25 independent fields. Fluorescence was visualized using a Nikon inverted microscope, or a vertical (E400) microscope, and images were captured with a Nikon digital camera.

### Example 5: Competition assay for the self-renewal effect of PEDF on neural stem cells in culture using the PEDF C-ter fragment.

The truncated form that encompasses the C-terminal half of the PEDF polypeptide antagonizes the action of full-length PEDF in the neural stem cell self-renewal assay, making it a useful tool for determining the specificity of the action of the full-length form (Figure 9). Thus, the current invention also provides a method for the analysis of the specificity of the effects of the PEDF molecule on the self-renewal process. To carry out the assay shown in figure 9, dissociated NSCs were seeded in complete medium with 20 ng/ml PEDF (which is equivalent to a 0.4 nM concentration) and in the presence of increasing concentrations of the C-ter PEDF fragment. After 4 DIV, the number of neurospheres formed in each condition was determined. As shown in figure 9, increasing concentrations of C-ter PEDF antagonize the PEDF effect, providing a specificity assay. The receptor for PEDF is still unknown although PEDF has been shown to interact with a membrane protein of approximately 80 kDa (Alberdi, E., Aymerich, M.S., Becerra, S.P. (1999) Binding of pigment epithelium-derived factor (PEDF) to retinoblastoma cells and cerebellar granule neurons. Evidence for a PEDF receptor. J. Biol. Chem. 274, 31605-31612*).* This type of assay suggests that the full-length and the truncated forms could be competing for the binding to a membrane receptor.

To determine if the truncated form is able to antagonize the effect of PEDF produced by mammalian cells, an assay was performed in COS cells, which do not synthesize significant amounts of PEDF. COS cells were transfected with a cDNA encoding human *PEDF.* To that end, the human Pedf (the gene's name in HUGO nomenclature is *Serpinf1*) cDNA was obtained by RT-PCR and was cloned in the pcDNA 3.1 vector (Invitrogen). Cells were co-tranfected with the aforementioned plasmid and with a GFP expression vector to visualize transfection, using Lipofectamine in DMEM. After 8 hours, cells were transferred to DMEM with 10% FBS for 12 additional hours. The expression and secretion of Pedf in cells transfected either with empty vector or pcDNA-hPedf was analyzed by RT-PCR and immunoblot of the conditioned media, respectively, as explained in the paragraphs that follow. Transfected cells were changed to NSC complete medium, an insert was placed (12 mm and 0.4 µm-pore diameter Millipore transwell) 2 hours later and dissociated neural stem cells were seeded at low density onto the insert. Cultures were grown in the absence or presence of C-ter PEDF at 15 nM concentration. The number of neurospheres formed in each culture condition was counted at 4 DIV. As shown in figure 10, NSC co-cultures with Pedf transfected COS cells displayed an increase in the number of neurospheres that was antagonized by the C-ter fragment. Figure 10 shows that COS cells transfected with a construct containing the human PEDF coding sequence express and secrete PEDF. This PEDF is active in a neural stem cell self-renewal assay and the effect is blocked by the C-ter fragment.

Thus, the C-ter PEDF truncated form allows to identify the participation of PEDF in a cellular system that expresses it by means of the specific antagonism of the truncated form, as shown in the following experiment, in which C-ter PEDF is used to antagonize the self-renewal effect exerted by cell types that naturally produce and secrete PEDF. For instance, figure 11 shows cell types that express PEDF, and among them, several ones that secrete detectable amounts of PEDF protein. These cell types are natural producers of the factor, such as endothelial cells or ependymal cells.

Several cell types were analyzed for these experiments:
- Arterial primary endothelial cells derived from human umbilical cord (HUAEC cells): obtained from AdvanceCell and maintained in medium recommended by the supplier.
- Venous primary endothelial cells derived from human umbilical cord (HUVEC cells): obtained from AdvanceCell and maintained in medium recommended by the supplier.
- Ependymal cells obtained following extraction of the ependymal layer of the adult mouse brain lateral ventricles and maintained in neurosphere complete medium.
- Astrocytic cells obtained following differentiation of neural stem cells in differentiation assays or obtained from astrocyte cultures derived from neonatal mouse brains.
- Granule neurons isolated from postnatal day 7 mouse cerebellum and seeded onto poly-D-lysine in DMEM-F12, 10% FBS and 25 mM potassium chloride.

These cell types were cultured either in their corresponding medium or in neurosphere complete medium. In order to analyze expression at the mRNA level, total RNAs were extracted with RNeasy Mini Kit (Qiagen) following the manufacturer's recommendations. 1 □g RNA was reverse transcribed into cDNA using random primers (3 □g/□L, Invitrogen Life Technologies, USA) and 200 U SuperScript II RT reverse transcriptase (Invitrogen Life Technologies) in a 40 □l reaction volume and in the presence of first-strand buffer (50 mM Tris-HCl, 75 mM KCl, 3 mM Mg₂Cl), 5 mM DTT and 0.25 mM each of the dNTPs (Amersham Pharmacia). PCR reactions were carried out in an Eppendorf thermocycler. As a template, 1 µl cDNA was used in a 20 µl reaction volume containing 4mM Tris-HCl, 20 mM KCl, 20 µM EDTA, 200 µM DTT, 0.25 mM dNTPs (Amersham Pharmacia), 0.25 µM primers (Sigma-Genosys) and 1 U DNA Taq polimerase (Eppendorf). After the initial denaturing step at 94°C for 2 min, the amplification cycles corresponding to each primer pair were carried out followed by a final extension at 72 °C for 10 min. The size of each amplification product was resolved by electrophoresis in 2% (p/v) agarose gels prepared in TBE buffer (89 mM Tris, pH 8.0, 89 mM Boric acid, 2mM EDTA) with 10µg/ml etidium bromide. A 50% glicerol, 0.05% bromophenol blue, 0.05% xylene cyanol, 100 mM EDTA solution was used as a 6x loading buffer. Quantification was carried out by densitometry and comparison of the intensity of each band with that of the amplification product of beta-actin gene. Primers used were: hPEDF (forward: CTGAAGCTGAGTTATGAAGGCG; reverse: GGTTAAGGTGATAGTCCAGCGC), hβ-actin (GCATGGAGTCCTGTGGCATCCACG; GGGTGTAACGCAACTAAGTCATAG), mPEDF (GAGCTACATCTTCTTCCTGCC;CCAGTAATCTTGCTGAAGTCGG),mβ-actin (CCGGGACCTGACAGACTACCT;GCCATCTCCTGCTCGAAGTCTA). For protein determination, either cells or medium conditioned by the cells was collected. Cells were homogeneized in RIPA lysis buffer (20 mM sodium phosphate buffer, 150 mM NaCl, 5 mM EDTA, 1% Triton X-100) supplemented with phosphatase and protease inhibitors (1 mM sodium orthovanadate, 1 mM sodium fluoride, 1 mM PMSF, 10 µglml aprotinin and 10 µg/ml leupeptin). The concentration of the protein that was extracted was determined by a modified Bradford method (DC Protein assay; Bio-Rad) using bovine serum albumin (BSA) as a standard. The same protein amount (50-100 µg) was resuspended in a sample dissolving buffer (625 mM Tris-HCl, pH 6.8, 10 %(v/v) glycerol, 2% (p/v) SDS, 4% (v/v) β-mercaptoethanol, 0.025% (p/v) bromophenol blue), was denatured by boiling for 5 minutes and was resolved by SDS-PAGE electrophoresis in 10-13% gels. Gels were electrotransferred (100 V, 1 hour) to a nitrocellulose membrane (Hybond-ECL, Amersham Biosciences), using transfer buffer (25mM Tris, pH 8.3, 20% (v/v) methanol, 192 mM glycine) in a Mini Trans-blot system (Bio-Rad). Membranes were blocked in 5% (p/v) fat-free powder milk prepared in tris borate saline-0.1% (v/v) Tween-20 (Sigma) (TBS-T, 0.1 M Tris-HCl pH 7.5, 0.9% (p/v) NaCl) and shaked during 1 hour at room temperature. Membranes were then incubated with anti-PEDF (Chemicon, 1:1000) and anti-beta-actin (Sigma; 1:5000) primary antibodies in TBS-T over night at 4 °C with shaking. Finally, membranes were washed in TBS-T, incubated for 1 hour at room temperature with the appropriate secondary antibodies bound to peroxidase and were developed by chemiluminiscence (Roche Lumi-light ECL kit) exposing the membranes to photographic films (Konica) until the optimal detection of the signal. The intensities of the individual bands were quantified using an image densitometer (Scion Image). The protein expression levels analyzed in our studies were normalized to β-actin from the same samples.

As shown in figure 12, the C-ter fragment has the ability to compete for the inductive effect of endothelial and ependymal cells in the neural stem cell self-renewal assay. This blockage is in accordance to the observation that these cell types secrete PEDF and demonstrates that PEDF naturally produced by certain cell types can be antagonized by the C-ter fragment in the self-renewal process. This is shown in figure 12. Figure 12 shows that endothelial and ependymal cells that naturally express and secrete PEDF (see figure 11) induce neurosphere formation and that this inductive effect is blocked by 15 nM C-ter PEDF. To carry out these experiments, the different cell types were seeded in 24-well plates with NSC complete medium, an insert was placed (12mm and 0.4 µm-pore diameter Millipore transwell) 2 hours later and dissociated neural stem cells were seeded at low density onto the insert. Cultures were grown in the absence or presence of C-ter PEDF at 15 nM concentration. The number of neurospheres formed in each culture condition was counted at 4 DIV. In NSC co-cultures with endothelial and ependymal cells, an increase in the number of neurospheres that was antagonized by the C-ter fragment was observed. In the astrocyte co-culture condition, there was an inductive effect in sphere formation that was not mediated by PEDF since it was not antagonized by the fragment (which is in accordance with the lack of PEDF secretion) and in the neuronal condition there was no effect. Altogether, this indicates that natural cell sources of PEDF can activate self-renewal and that the C-ter fragment can be utilized to demonstrate the specificity of such effect.

In order to demonstrate in parallel that endothelial cells favor neural stem cell self-renewal due to PEDF production, endogenous PEDF expression was silenced in these cells using RNA interference techniques, and a reduction in the inductive effect of HUVEC cells on sphere formation was reduced when endogenous expression and secretion of PEDF was diminished. This is shown in figure 13. Therefore, figure 13 shows that HUVEC cells with partial silencing of PEDF expression using a specific siRNA secrete less PEDF to the medium and induce the formation of a smaller number of neurospheres than HUVEC cells treated with a control siRNA. To do this experiment we got specific 21-bp siRNAs from PROLIGO (siPedf 5'-CGAGUUCAUUCAUGACAUAGA-3'. Sicontrol (luciferase) 5'-AACGUACGCGGAAUACAACGA-3') and we introduced them in the cells by two rounds of transfection with Lipofectamine (Invitrogen). We determined the levels of PEDF in the growth media by inmunoblot and the sphere number by co-culture, as it was explained previously.

### Example 6: Self-renewal assay of neural stem cells in the neurogenic niche of the subventricular zone by means of intra-cerebral PEDF delivery

PEDF is also able to activate the self-renewal division of neural stem cells present in the endogenous neurogenic niches. It increases their number and their capacity to generate a progeny but keeping intact their stem cell state (self-renewal), as it is demonstrated by the intra-cerebral PEDF delivery with mini-osmotic pumps in mice brains (Figures 14 and 15). As shown in Figure 14 PEDF induces a bigger number of neural stem cells (that retain BrdU labeling longer times, as an indication of their relative quiescent state, and that are positive for the marker GFAP) when it is injected in the brain. In normal conditions the blockade of endogenous PEDF in the neurogenic niche of the subventricular zone does not produce any change, suggesting that PEDF is not acting as a survival factor for these cells.

Figure 15 shows that the effect of the PEDF administration in mouse subventricular zone is to activate the neural stem cell division, as it is reflected by the increase in BrdU incorporation, an indication that they are proliferating more in the presence of exogenous PEDF. To fulfill these experiments Alzet mini-osmotic pumps (model 1007D) were attached to a 28-µm gauge cannula and implanted intra-cerebrally in adult female mice. The stereotaxic coordinates related to Bregma were 0.0 anteroposterior, 0.7 medilateral and 1.7 dorsoventral. The cannula was sealed to the skull with Hystoacryl cement (B/Braun). Saline solution (vehicle), PEDF (20ng/ml) or C-ter PEDF (100nglml) were infused during 7 days at a 0.5 µl/h rate. To analyze the different proliferative cell populations in the subventricular zone, including neural stem cells, we used BrdU incorporation technique. BrdU is an analog of thymidine, and incorporates into the DNA of cells in S phase in the moment of exposure to the nucleotide. After that we can detect the labeled cells by means of inmunodetection with specific antibodies in histological sections. Depending on the proliferating cells to mark in the adult brain, mice received two kinds of BrdU administration regimes:
- one set of mice received 7 BrdU injections intraperitoneally the last day of the infusion period. BrdU (Sigma) was injected every two hours (Nowakowski et al., 1988) at 50□g/g of animal weight, and were sacrificed one hour after the last injection. This strategy labels cells proliferating the last day of infusion.
- another set of mice received 6 BrdU injections in two days (3x2) just before the pump implantation and were sacrificed at the end of the infusion period. This strategy label neural stem cells that are relatively quiescent and retain the mark associated with BrdU incorporation during one month. These cells express GFAP.

All the animals were anesthetized with pentobarbital sodium (13mg/g of animal) and sacrificed by intracardial perfusion of paraformaldehyde (PFA) 4% (w/v) in phosphate buffer 0.1M, pH 7.4 (PB). After overnight post-fixation brains were dissected and washed 2 hours in PB, dehydrated and embedded in paraffin. 7 mm serial sections were incubated 20 min in HCl 2N at 37 C and neutralized with borate buffer (0.1M, pH 8.5). Non-specific binding was avoided by incubating the sections with a blocking buffer (0.2% Triton X-100, 10% goat serum in PB 0.1M) for 1 hour at room temperature. Samples were incubated for 12h with the primary antibody against BrdU (DAKO, 1:200) diluted in blocking buffer, followed by incubation with fluorochrome labeled secondary antibodies. This inmunocytochemical detection was combined with the inmunodetection of neural stem cells specific markers, like GFAP (glial fibrillary acidic protein), and neuroblasts, like PSA-NCAM (polysialated acid-neural cell adhesion molecule).

PEDF increases the efficiency of the niche to produce a neuronal progeny (Figure 16). Figure 16 shows that exogenous PEDF administration induces neuronal production in the subventricular zone (stained with PSA-NCAM antibody) and increases also the proliferative activity in the area (BrdU incorporation). The opposite effect is seen when the endogenous PEDF is blocked by the inactive fragment C-ter PEDF. Moreover PEDF administration induces the production of cell cultures with more neurosphere forming capacity (Figure 17). In the Figure 17 it is shown how more neurosphere production is achieved from tissue dissociated from exogenously infused PEDF brains. This indicates that PEDF activation of somatic stem cells favors the production of cellular progeny so it can contribute to regenerative and tissue repairing processes.

### Example 7: Assays related with PEDF sources

Four examples of PEDF production are included in order to demonstrate that the main object of the present invention is the new use of PEDF factor as an inducer of self-renewal in stem cells, independently of the way that PEDF had been obtained:
- From naturally expressing and producing PEDF cells, like for example, endothelial cells (for example, umbilical cord) or cells of ependymary nature (see Figure 11). Those cells secrete PEDF when cultured in their regular cell culture media or when cultured in neural stem cell media. They can be primary cultures or cell lines of endothelial or ependimary origin, handed over or acquired from companies. In order to use them as a PEDF source for self-renewal they can be co-cultured with stem cells by means of inserts for trans-membrane co-cultures that do not allow contact between the two types of cells, as it was explained for the assays in example 5.
- From cells that express heterologously the PEDF sequence, like for example COS cells. For that PEDF can be expressed in any cell line that does not express or express very low levels of PEDF by introducing a cDNA for Pedf using transfection, like it is explained for example in the assays included in the example 5.
- As a recombinant protein in bacteria (*E*. *coli*)*.* We have demonstrated that when produced in bacteria PEDF has also effect in the self-renewal capacity of neural stem cells: 41±1% more neurospheres in PEDF treated cultures, n = 3; p ≤ 0.01, Student's t-test. This PEDF was acquired from Bioproducts MD.
- Produced in *Pichia pastoris*: from the human sequence obtained by PCR and cloned into pPICZαA with the addition of a signal peptide from *Saccharomyces cerevisiae* and a histidine tail. Once produced in *Pichia,* the PEDF is purified by chromatography. The efficiency and purity of the production is tested by SDS-PAGE electrophoresis and by western-blot with specific antibodies against PEDF (see Figure 18). These molecules were obtained in the lab of Julio Escribano at the University of Castilla La Mancha, and were provided to us for the analysis shown in the examples. Figure 18 shows the forms purified by chromatography of complete PEDF and the C-terminal half of human PEDF obtained both from genetically modified *Pichia* (over-expressing those forms). The SDS-PAGE electrophoresis shows the purity of the extraction and the apparent molecular weight of PEDF (47kDa) and C-ter PEDF (26kDa) and the inmunodetection with antibodies against PEDF shows the specificity of both products.

In the context of this invention, the use of biologically active PEDF in the self-renewal process of stem cells includes PEDF and any PEDF derivative with effect in self-renewal. PEDF can be purified from natural sources, like the vitreous. The invention is not restricted to the use of the complete form of PEDF or to the precise sequence or to its origin from humans or other species. A codifying sequence for PEDF can include populational allelic variations or point mutations, as well as variations produced by insertions, deletions, or substitutions of PEDF polypeptides found in nature. A PEDF polypeptide may contain other domains, like special epitope tags for purification (for example, histidine tails, myc) or for tracing (for example, GFP). They can be variations by post-translational modifications, like glycosilations or phosphorylations. PEDF can be used in its complete form or as fragments that retain its activity over self-renewal. All these forms can be administered directly to stem cell cultures for their activation and maintenance. They can be also applied systemically or locally using pharmaceutical compositions for self-renewal characterized by containing at least a pharmaceutically effective quantity of PEDF factor and at least an acceptable pharmaceutical medium. Related to this, PEDF is not toxic and we have found effects in self-renewal, at least in mouse neural stem cells, both in cell culture experiments and in direct mouse brain administration, with concentrations as low as 0.4 nM and 400 nM, respectively.

PEDF can be used as a construct containing a nucleic acid sequence codifying any of the forms mentioned in the previous paragraph. In this case, a coding sequence can be introduced in cellular systems by means of DNA recombinant technology. PEDF can be introduced in this way in cellular systems (by transfection, infection, electroporation, microinjection,...) that, afterwards, can be placed in co-culture with stem cells, or introduced in tissues where the activation of endogenous stem cells is needed, i. e. by infection with viral vectors/viruses used in gene therapy. These sequences can also be used for the production of an active form of PEDF in self-renewal in prokaryotic or eukaryotic expression systems. In all these cases, the constructs must contain promoters able to direct the expression of the molecule in the cells of interest. In all cases, PEDF can be administered alone or in combination with other factors that induce self-renewal. In the context of the present invention we also propose the use of C-terminal fragments of the PEDF molecule of various lengths as a strategy to block the self-renewal effect induced by the full length PEDF.

The PEDF polypeptide activates the self-renewing division of somatic stem cells, i. e. neural, in the natural niches and, therefore, the production of differentiated progeny. The present invention provides a method to activate stem cells present in those tissues that require a higher level of stem cell activity for tissue renewal and turnover and even for regeneration, like for example skin or hematopoyetic system regeneration, neural regeneration after stroke, heart regeneration after ischemia/infarct, etc.

## Claims

1. Use of the PEDF factor, in its complete or fragmented sequence, for enhancing symmetrical self-renewal of stem cells cultured *in vitro.*

2. Use, according to the claim 1, wherein the stem cells are adult cells or embryonic cells.

3. Use, according to claim 1, wherein the stem cells are native or genetically / epigenetically modified stem cells.

4. Use of the PEDF factor, in its complete or fragmented sequence, for manufacturing medicaments for the regeneration treatment of cardiac, skin, neural and hematopoietic tissues.

5. Use, according to claim 4, wherein the medicaments additionally comprise molecules selected from: amino acids, lipids, carbohydrates and metals.

6. Use, according to claims 4 or 5, wherein the medicaments additionally comprise a pharmaceutically acceptable carrier.

7. Use, according to claims 4 to 6, wherein the medicaments are in the form selected from: powder, granulates, tablets, capsules, troches, solution, suspension, emulsion and syrup.

8. Use, according to any of the preceding claims, **characterized in that** the PEDF factor quantity is at least 20 ng/ml.

9. Method of tissue repair comprising transplants of stem cells cultured *in vitro* in the presence of PEDF factor.

10. Method, according to claim 9, wherein the tissue to be repaired is selected from: skin tissue, cardiac tissue, haematopoietic tissue or neural tissue.

## Patentansprüche

1. Verwendung des PEDF-Faktors in seiner vollständigen oder fragmentierten Sequenz zur Verbesserung der symmetrischen Selbsterneuerung von in vitro kultivierten Stammzellen.

2. Verwendung nach Anspruch 1, wobei die Stammzellen adulte Zellen oder embryonale Zellen sind.

3. Verwendung nach Anspruch 1, wobei die Stammzellen ursprüngliche oder genetisch / epigenetisch veränderte Stammzellen sind.

4. Verwendung des PEDF-Faktors in seiner vollständigen oder fragmentierten Sequenz zur Herstellung von Medikamenten für die Regenerationsbehandlung von Herz-, Haut-, Nerven- und hämatopoetischen Gewebe.

5. Verwendung nach Anspruch 4, wobei die Medikamente zusätzlich Moleküle umfassen, die ausgewählt sind aus: Aminosäuren, Lipide, Kohlenhydrate und Metalle.

6. Verwendung nach Anspruch 4 oder 5, wobei die Medikamente zusätzlich einen pharmazeutisch akzeptablen Träger umfassen.

7. Verwendung nach Anspruch 4 bis 6, wobei die Medikamente in einer Form vorliegt, die ausgewählt ist aus: Pulver, Granulat, Tabletten, Kapseln, runde Tabletten, Lösung, Suspension, Emulsion und Sirup.

8. Verwendung nach einem der vorhergehenden Ansprüche, **dadurch gekennzeichnet, dass** die Menge des PEDF-Faktors mindestens 20 ng/ml beträgt.

9. Verfahren der Gewebereparatur, welches die Transplantation von in vitro kultivierten Stammzellen in Anwesenheit des PEDF-Faktors umfasst.

10. Verfahren nach Anspruch 9, wobei das zu reparierende Gewebe ausgewählt ist aus: Hautgewebe, Herzgewebe, hämatopoetisches Gewebe und Nervengewebe.

## Revendications

1. Utilisation du facteur PEDF, dans sa séquence complète ou fragmentée, pour accroître l'autorenouvellement symétrique de cellules souches cultivées *in vitro.*

2. Utilisation, selon la revendication 1, dans laquelle les cellules souches sont des cellules adultes ou des cellules embryonnaires.

3. Utilisation, selon la revendication 1, dans laquelle les cellules souches sont des cellules souches naturelles ou génétiquement / épigénétiquement modifiées.

4. Utilisation du facteur PEDF, dans sa séquence complète ou fragmentée, pour fabriquer des médicaments destinés au traitement de régénération des tissus cardiaque, cutané, nerveux et hématopoïétique.

5. Utilisation, selon la revendication 4, dans laquelle les médicaments comprennent en outre des molécules sélectionnées parmi : acides aminés, lipides, hydrates de carbone et métaux.

6. Utilisation, selon les revendications 4 ou 5, dans laquelle les médicaments comprennent en outre un vecteur de qualité pharmaceutique.

7. Utilisation, selon les revendications 4 à 6, dans laquelle les médicaments se présentent sous la forme sélectionnée parmi : poudre, granulés, comprimés, capsules, trochisques, solution, suspension, émulsion et sirop.

8. Utilisation, selon n'importe laquelle des revendications précédentes, **caractérisée en ce que** la quantité de facteur PEDF est au moins de 20 ng/ml.

9. Procédé de réparation tissulaire comprenant des greffes de cellules souches cultivées in vitro en présence du facteur PEDF.

10. Procédé, selon la revendication 9, dans lequel le tissu à réparer est sélectionné parmi : tissu cutané, tissu cardiaque, tissu hématopoïétique ou tissu nerveux.
